# EUROPEAN PATENT APPLICATION

(11) **EP 2 821 785 A1**
(43) Date of publication of application: **07.01.2015**
(21) Application number: 14425090.9
(22) Date of filing: 04.07.2014
(51) Int. Cl.: G01N 33/00

(54) **Device for measuring concentration of gases**

(30) Priority: 05.07.2013 IT RM20130395
(71) Applicant: Universita' Degli Studi Di L'Aquila, 67100 L'Aquila (IT); Universita' Degli Studi "G. d'Annunzio" Chieti-Pescara, 66013 Chieti (IT)
(72) Inventor: Rosatelli, Gianluigi, 66013 Chieti Scalo (Chieti) (IT); Di Carlo, Piero, 67100 L'Aquila, AQ (IT); Giammaria, Franco, 67100 L'Aquila, AQ (IT); Aruffo, Eleonora, 67100 L'Aquila, AQ (IT); DEL Grande, Francesco, 67100 L'Aquila, AQ (IT); Visconti, Guido, 67100 L'Aquila, AQ (IT); Stoppa, Francesco, 66013 Chieti Scalo (Chieti) (IT)
(74) Representative: Iannone, Carlo Luigi

(57) **Abstract**

The invention concerns a device 1) for measuring concentration of gases, particularly trace gases, comprising, within an housing (2) having such reduced dimensions to allow its introduction within a well or a piezometer, very small sized concentration meters, or sensors, for different types of gaseous chemical species and weather parameters, a real time calibration system, after every acquisition, of the different sensors to take into consideration the variations of all the environmental parameters, and a GPS system allowing synchronizing measurements carried out by other measurement devices such as, for example, accelerometers and seismographs.

## Description

The present invention refers to a device for measuring concentration of gases, particularly trace gases.

More particularly, the invention concerns the realization of a multiparameter miniaturized device, for synchronous measure, in situ, of concentrations of trace gases (CO₂, CO, CH₄, O₃, NH₃) and weather parameters (temperature, pressure, humidity) naturally released from the terrestrial crust.

As it is known, according to scientific literature (see, for example, Cicero et al., 2009) it is pointed out that the phenomena preceding disastrous seismic events are multiple, and in particular can be seismic *foreshocks,* variation of the natural gas flow from the ground (e.g. CO₂, He, Rn), local variations of the magnetic field, variations of the level of the water-bearings, change of electrical resistance of rocks, emission of anomalous radio waves, and others.

These phenomena are generally dependent on the deformations occurring within the terrestrial crust during a seismic crisis culminating in a seismic event.

However, chemical/physical mechanisms resulting in the instantaneous seismic energy release (earthquake) during a seismic crisis are unknown.

Up to now tests relating to what happens during an earthquake have been known: the terrestrial crust is cracked resulting in a relative movement between two portions of crust. This process increases the fracture along the zone wherein the movement occurs (fault). This results in an increase of the rock permeability due to microfractures being propagated before the movement along the fault occurs, thus releasing seismic energy in form of elastic waves. The increase of the permeability favors the flow towards the surface of gases trapped in the crust and/or upwardly moving from the terrestrial mantle.

Where the terrestrial crust consists of carbonated rocks, the tension building up in proximity of the fault zone results in the de-calcination of the carbonated rocks thus producing a release a lot of CO₂.

In particular structural geological arrangements (like those in central Apennines), the rock fracturing seems to favor the surface transfer of gas (e.g. CO₂, He) from the mantle depths. In the last seismic crises resulting in remarkable destruction damages in L'Aquila and between Umbria and Marche hearth-quake zones, geochemical studies of the compositions of the gaseous phases dissolved in layer waters, have put in evidence variations both of the content of dissolved CO₂ and isotopic composition of C and He.

Currently, all the measures of gaseous compounds emitted from the subsoil are made by the analysis of species dissolved in water, or by cabinets for the analysis of a single gas, like CO₂, very more expensive and cumbersome. Other systems for the measure of more than one gaseous species at the same time imply the collection of gas samples from the soil and laboratory analysis thereof, thus not allowing a continuous measurement.

Based on the above, the Applicants have realized and set up a measuring device for the monitoring of the concentrations of gases released in zones where there are present active faults in order to verify the possibility to use the variation of the concentration of some spy gases, naturally emitted from the terrestrial crust like premonitory marker of seismic events.

In particular, the solution proposed according to the present invention is devised for ad hoc constructed o pre-existing underground wells, providing also for a GPS system allowing to synchronize the carried out measures with other measuring devices like for example accelerometers and seismographs.

The solution according to the present invention allows the analysis management and the synchronization with other measuring devices is managed by a specific software processing also the measures of the chemical-physical parameters in continuous, memorizes and transmits in real time the data to a local server, and manages the auto-calibration of the individual sensors. It is therefore a specific object of the present invention a device for measuring concentration of gases, particularly trace gases, comprising, within an housing (2) having such reduced dimensions to allow its introduction within a well or a piezometer, very small sized concentration meters, or sensors, for different types of gaseous chemical species and weather parameters, a real time calibration system, after every acquisition, of the different sensors to take into consideration the variations of all the environmental parameters, and a GPS system allowing synchronizing measurements carried out by other measurement devices, such as, for example, accelerometers and seismographs.

In particular, according to the invention, said sensors measure concentration of CO₂, CO, CH₄, O₃, NH₃, SO₂ and temperature, pressure and humidity.

Further, according to the invention, said sensors are electrolytic, resistive and solid state sensors.

Again according to the invention, concentration ranges that can be measured are 100 - 4000 ppm (parts per million) for CO₂, 30 - 1000 ppm for CO, 500 - 10000 ppm for CH₄, 20 - 200 ppm for O₃ and 30 - 300 ppm for NH₃.

Further according to the invention, said device is provided with a remote data transmission system, such as a wi-fi or remote-gprs system..

Again according to the invention, said device provides for a specific software managing continuously measurements of chemical-physical parameters, stores and transmits in real time said data to a local server, and manages calibration of single sensors.

In particular, the software permits acquisition of data of device, as well as self-calibration, by acquisition and transmission of data obtained in real time, and the possibility of remote and in real time interrogating each one of device sensors to verify correct operation and extraordinary calibration processes, by placing side by side within a standard gas well.

Again according to the invention, said device provides for a digitalization of signal close to the sensors and surface transfer of data by a quick and reliable protocol.

The present invention now will be described, by an illustrative, but non limitative way, with particular reference to preferred embodiments thereof, illustrated in the figures of the attached drawings, wherein:

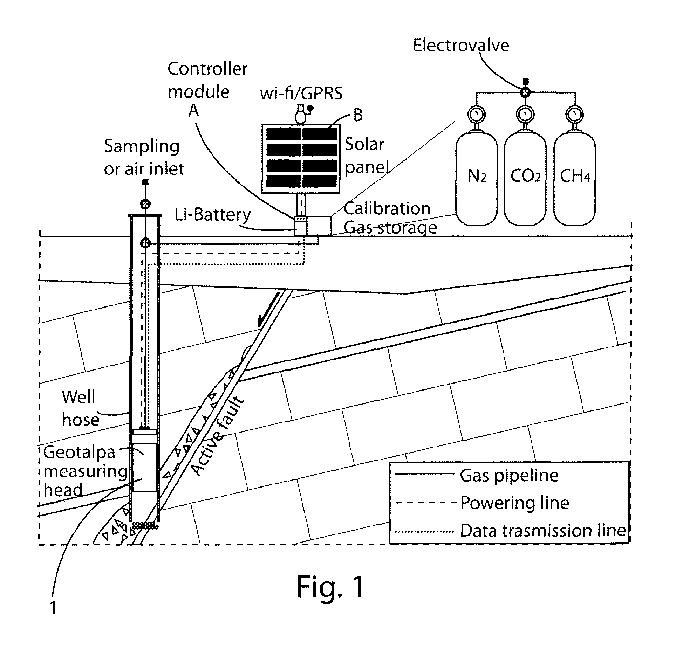

figure 1 is a first perspective view of the device according to the invention;

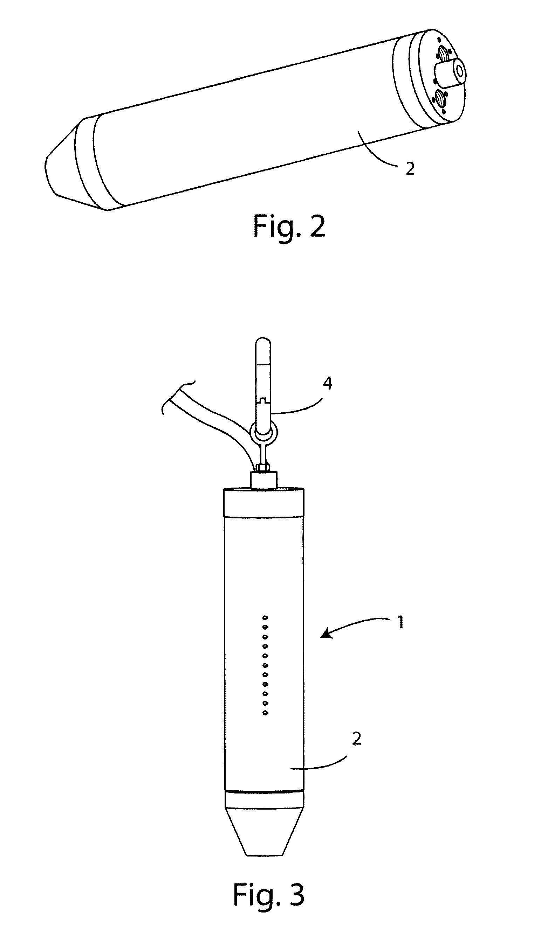

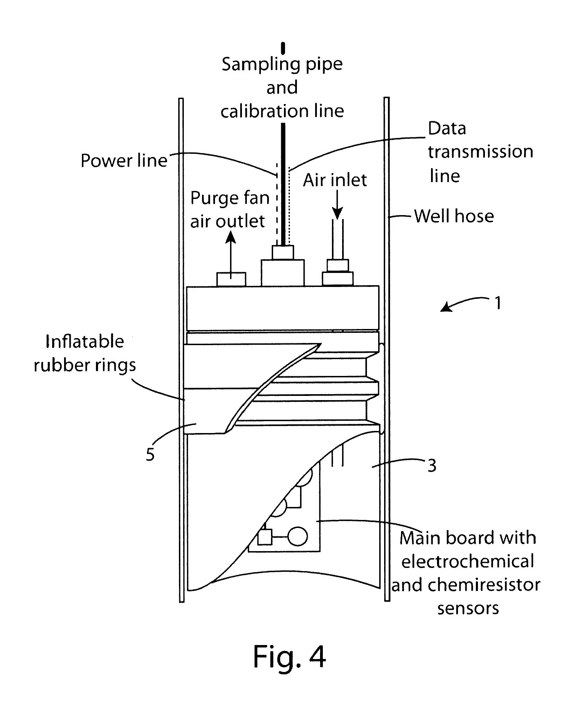

figure 2 is a second view of the device according to the invention; and
figure 3 shows the inside of the device of the previous drawings.

In the enclosed figures a very small device 1 according to the invention, providing for an housing 2, is illustrated, inside of which is provided for a card whereon there are sensors, again extremely reduced in size, for the measurement of concentration of several types of gaseous chemical species and weather parameters. Said housing 2 is equipped, in addition, with means 4 for the connection and insertion in a well. The device is connected, through a cable, to a surface arranged compact system for data acquisition and transfer and a solar panel for the power feeding.

The device 1 according to the invention is equipped with very small sized electrolytic, resistive and solid state meters of concentration of several types of chemical gaseous species (CO₂, CO, CH₄, meteorological O₃, NH₃, SO₂) and weather parameters (temperature, pressure and humidity), in such a way that, as a whole, it can be installed in reduced size housings, for example 77 mm x 100 mm, and inserted in wells crossing the more permeable fault zone.

The ranges of measurable concentrations are: 100 - 4000 ppm (parts per million) for CO₂, 30 - 1000 ppm for CO, 500 10000 ppm for CH₄, 20 - 200 ppb for O₃ and 30 - 300 ppm for NH₃.

These concentration ranges are theoretical ones typical for subsoil natural outflow. The device 1 according to the invention is suitable to send, using wi-fi or remote-gprs technology, the measurement data collected at time intervals of the order of 2 second to a remote PC, where the operator can control the variations of the gas flow occurring in correspondence of the fault.

Moreover, device 1, through GPS, is synchronized both with the seismographs of the national monitoring network and local networks. This allows to integrate the measures of gases with other measuring apparatuses (magnetometers, ultrasound, resistance meters, etc.), possibly installed on the same fault zone.

The synchronization of various measuring devices 1 allows to correlate in real time both seismic tremor and more energetic seismic shocks with the composition variation of the main gases released from crust rocks and other parameters possibly under measurement.

Using the measuring device 1 according to the invention therefore it is possible to detect whether there is a correspondence between the variation of gas concentration and the seismic activity of fault whereon it is installed and possibly other monitored chemical-physical parameters. In positive correlation case between event and gas release, it is possible to use, in principle, the measure of concentration of gases released along the fault as marker of the occurrence of an important seismic event.

Moreover, the continuous monitoring and the collected data can help to comprise the chemical-physical mechanisms by which a seismic event is developed.

The possibility to install easily the device according to the invention in a well allows to have a reduced influence on the measurements by the contribution due to the atmosphere or biological processes occurring in subsoil, thus the obtained measurements are directly usable without the need for carrying out difficulty evaluable corrections.

By using the device 1 according to the invention it is possible to measure, at the same time, in situ, in continuous way for long periods of time six gaseous phase compounds (the time scale for the development of a seismic crisis until the main event in Apennines is variable from 1 to 3 years).

Moreover the reduced dimensions make it ideal in order to be installed along fault zones with low costs and very short times.

The device according to the invention moreover is equipped with an auto-calibration system in real time, after every acquisition, of the various sensors in order to evaluate the variations of all the environmental parameters (pressure, temperature and humidity). The auto-calibration system is based on a laboratory characterization of the sensors, since the response thereof depends on the variations of temperature and atmospheric humidity, and the application of a specific software calibrating the response of the individual sensors with the variation of the atmospheric parameters.

Another particularity of the device 1 according to the invention consists of the signal digitalization in proximity of the sensors and the surface transfer of the data using a fast and reliable protocol. This allows to be able the device 1 to be arranged also at remarkable distance (various hundred of meters) from the system of data acquisition, without decrease of the sensor performances. The logistic flexibility of the device 1 according to the invention make it usable also in particularly uneven areas.

Further peculiarity of the solution proposed according to the invention consists of the joining to the device 1 of a calibration system for each sensor with sample gas for the in situ periodic calibration without necessity the device to be removed from the subsoil.

The device 1 according to the invention can be installed also in spy wells and piezometers (at heights greater than the maximum layer excursion) placed within and out of landfill bodies in order to monitor the polluting gases released through the fermentation of the organic substances (mainly CO₂, CH₄).

The device according to the invention can be particularly interesting for monitoring gases released underneath the terrestrial surface in correspondence of areas used for the storage of dangerous materials or areas used for storage of atmospheric CO₂, in order to control and abatement of the environmental CO₂.

A further application of the device according to the invention concerns the monitoring of oil and natural gas wells and all the activities involving the perforation of the terrestrial crust.

Moreover in the mines wherein coal or other materials are extracted, accidental explosions due to sudden release of CH₄ (firedamp) or episodes of anoxia by O₂ reduction and CO₂ increase resulting from oxidation of buried organic substance can occur. The device according to the invention could be applied also for the prevention of accidents also in these contexts, or for the study and monitoring of greenhouse gases like those released from permafrost.

At last, the possibility of using the device according to the invention for the monitoring of the air quality must be mentioned.

In fact, being of small dimensions and requiring low energy for operation thereof, it could be installed also on fixed supports like poles of the street lighting system. Having moreover a very low realization cost (approximately 20 times less than a standard control unit for the monitoring of the air quality), it is surely competitive also for the realization of city control networks.

The present invention has been described by an illustrative, but not limitative way, according to preferred embodiments thereof, but it is to be understood that variations and/or modifications can be carried out by those skilled in the art without thus departing from the scope thereof as defined in the enclosed claims.

## Claims

1. Device (1) for measuring concentration of gases, particularly trace gases, comprising, within an housing (2) having such reduced dimensions to allow its introduction within a well or a piezometer, very small sized concentration meters, or sensors, for different types of gaseous chemical species and weather parameters, a real time calibration system, after every acquisition, by the different sensors to take into consideration variations of all the environmental parameters, and a GPS system permitting synchronizing measurements carried out by other measurement devices such as, for example, accelerometers and seismographs.

2. Device (1) according to claim 1, wherein said sensors measure concentration of CO₂, CO, CH₄, O₃, NH₃, SO₂ and temperature, pressure and humidity.

3. Device (1) according to anyone of the preceding claims, wherein said sensors are electrolytic, resistive and solid state sensors.

4. Device (1) according to anyone of the preceding claims, wherein concentration ranges that can be measured are 100 - 4000 ppm (parts per million) for CO₂, 30 - 1000 ppm for CO, 500 - 10000 ppm for CH₄, 20 - 200 ppm for O₃ and 30 - 300 ppm for NH₃.

5. Device (1) according to anyone of the preceding claims, wherein said device is equipped with a remote data transmission system, such as a wi-fi or remote-gprs system.

6. Device (1) according to anyone of the preceding claims, wherein said device provides for a specific software managing continuously measurements of chemical-physical parameters, stores and transmits in real time said data to a local server, and manages calibration of single sensors.

7. Device (1) according to claim 6, wherein said software permits acquisition of data of device, as well as self-calibration thereof, by acquisition and transmission of in real time obtained data and possibility of remote and in real time interrogating each one of device sensors to verify correct operation thereof and extraordinary calibration processes, by placing side by side within a standard gas well.

8. Device (1) according to anyone of the preceding claims, wherein said device provides for a digitalization of signal close to the sensors and surface transfer of data by a quick and reliable protocol.
